# EUROPEAN PATENT SPECIFICATION

(11) **EP 2 683 235 B1**
(45) Date of publication and mention of the grant of the patent: **31.12.2014**
(21) Application number: 11706831.2
(22) Date of filing: 08.03.2011
(51) Int. Cl.: A01N 25/16, A01N 25/30, A01N 31/02, A01N 41/02, A47K 5/14, A61Q 19/10, A61K 8/04

(54) **LOW ALCOHOL ANTIMICROBIAL CLEANSING COMPOSITION**
ANTIMIKROBIELLE REINIGUNGSZUBEREITUNG MIT WENIG ALKOHOL
COMPOSITION NETTOYANTE ANTIMICROBIENNE PAUVRE EN ALCOOL

(43) Date of publication of application: 15.01.2014
(73) Proprietor: Sca Hygiene Products AB, 405 03 Göteborg (SE)
(72) Inventor: HEDBOM, Torsten, 791 29 Falun (SE); SNELL, Stefan, S-79141 Falun (SE)
(74) Representative: Hoffmann Eitle
(86) International application number: PCT/EP2011/053448
(87) International publication number: WO 2012/119644

(56) References cited:
- EP-A1- 0 384 126
- EP-A1- 0 640 285
- EP-A1- 1 129 702
- EP-A2- 1 967 066
- WO-A1-93/07250
- WO-A1-2008/003779
- WO-A1-2008/132621
- US-A1- 2009 236 371

## Description

The present invention relates to an antimicrobial cleansing composition which can be used to generate cleansing foam and which has a high antimicrobial efficacy despite the relatively low alcohol content.

### Technical background

In hospitals and doctors' practices, it is of utmost importance to work in a germ-free environment when patients are treated. For this reason, physicians, nurses and other medical staff are obligated to frequently wash and disinfect their hands. Customarily, this is done by first washing and cleansing the hands using a detergent-based liquid soap followed by a second wash involving the wetting and rubbing of the hands with a disinfectant composition. In hospitals and doctors' practices, there are typically two dispensers: one for the liquid soap and one for the antibacterial composition, which are mounted to the wall above the wash basins.

As a rule, commercially available disinfectant compositions are alcohol-based and contain ethanol and/or isopropyl alcohol in amounts of more than 60% by weight. It is generally believed in the art that these compositions require a fairly high alcohol content to be antibacterial. However, since long term use of high ethanol disinfectant compositions can be associated with skin irritation, there is a need for disinfectant compositions having a lower alcohol content. Furthermore, it would be desirable to reduce the time and effort required for cleansing and disinfecting the hands in hospitals and doctors' practices.

US 2002/007257 A1 discloses a sanitizing hand cleanser comprising approximately 60 wt.% to approximately 90 wt.% of one or more organic alcohols, approximately 5 wt.% to approximately 35 wt.% of one or more silicone-based materials, one or more thickening agents and optional humectants and skin pH-maintaining additives.

WO 2008/003779 A1 concerns ready-to-use skin friendly, broad spectrum disinfectant compositions which include both an alcohol and hydrogen peroxide as active disinfecting constituents. The compositions are aqueous compositions for providing a foam, comprising: 0.1-5% (w/w) hydrogen peroxide, 21-55% (w/w) of a C2-C6 alcohol, and 0.01-2% (w/w) of a foam booster.

EP 0 640 285 A1 relates to a washing agent for disinfecting and decontaminating hands, which is characterized in that it comprises an aqueous solution containing a) 5 to 35 % by weight of C1-C19-alkyl alcohol, b) 0.1 to 10.0 % by weight of naturally occurring C6-aryl-C1-C8-alkyl alcohol, c) 0.5 to 45 % by weight of sulphosuccinate, betaine or fatty alcohol ether sulphate, or a mixture of these, and d) 0.1 to 5.0 % by weight of dermatologically acceptable alpha-hydroxycarboxylic acid as skin protection component and for bringing the pH to 2 to 7.

EP 1 129 702 A1 relates to cosmetic compositions in the form of a shower gel in a pressurised container. The pressurised container contains a shower gel composition, said composition comprising suitable shower gel components and, based on the weight of the composition, at least 40 % of a low boiling, pressurised gas. In the container at least one alcohol is present in an amount of from 8 to 20 wt.% of the composition, wherein said alcohol is selected from the group of ethanol, isopropanol, n-propanol, n-butanol, t-butanol, iso-butanol, and mixtures of two or more of these.

EP 1 967 066 A2 relates to an alcohol containing agent (I) which shows a micro-biological effectivity within a period of less than 30 seconds, for the fast disinfection of dead surfaces, is claimed. The alcohol is selected from ethanol and n-propanol and iso-propanol. The alcohol containing agent additionally contains an amphoteric surfactant.

EP 0 384 126 A1 relates to a sprayable surface disinfectant based on low aliphatic alcohols and anionic surfactants and usual additives such as corrosion inhibitors, dyes and perfumes, characterized by the combination of: a) 20 to 35 wt.% of a mixture of ethyl alcohol and isopropyl alcohol in a weight ratio of 1:2 to 2:1, b) 0.05 to 0.5 wt.% of a mixture of anionic surfactants consisting of b-1) 0.04 to 0.4 wt.% of a primary or secondary alkane sulphonate or alkyl sulphate with 10 to 18 C atoms in the alkyl group and b-2) 0.01 to 0.1 wt.% of an alkyl ether sulphate with 10 to 14 C atoms in the alkyl group in the form of the sodium, magnesium or mono- or triethanol ammonium salt, the weight ratio of alkanol sulphonates and alkyl sulphates (b-1) to alkyl ether sulphates (b-2) being in the range of 10:1 to 2:1, the weight ratio of the total alcohol content (a) to total surfactant content (b) being in a range of 300:1 to 50:1, c) an acidification agent for the setting of a pH value from 2 to 6 or an alkalization agent for the setting of a pH value from 8 to 12, whilst d) the remainder is water.

WO 93/07250 A1 relates to a process for antiseptically cleaning the skin and hands and compositions therefor. The antiseptic cleansing composition comprises an alcohol and a surfactant, wherein the alcohol is selected from ethanol, n-propanol and isopropanol in a concentration of 40-80% of the total volume. The surfactant is selected from the group consisting of an anionic, a non-ionic and an amphoteric surfactant.

WO 2008/132621 A1 concerns a foamable sanitizing composition comprising: an alcohol, the alcohol being present in the composition in an amount of at least 20 percent by weight; a foaming agent; and a foam strengthening agent comprising an amphoteric or zwitterionic surfactant containing surfactant molecules in which at least 90% of the molecules have a hydrophobic carbon chain length of 18 carbon atoms or greater.

It is one object of the present invention to provide an alcohol-based composition which has good antimicrobial and cleansing properties.

It is a further object of the present invention to provide an antimicrobial cleansing composition which does not irritate the skin.

According to one embodiment of the present invention, it is an object to provide an antimicrobial cleansing composition able to be dispensed as a sufficiently stable foam.

Further objects and benefits of the present invention will become apparent from the following description of the invention and its embodiments.

Whenever the present description and the claims define a composition as "comprising" certain ingredients, the respective composition may also "consist of" these ingredients in a further embodiment of the present invention. If the terms "optionally" or "may" are used, this indicates that the corresponding ingredient can also be absent from the respective composition. Hereinafter, the unit "wt.%" refers to the total weight of the antimicrobial cleansing composition.

### Summary of the present invention

The present invention provides an antimicrobial cleansing composition comprising
(a) 20 to less than 60 wt.%, preferably 25 to 55 wt.%, more preferably 35 to 50 wt.% of ethanol containing not more than 20 wt.% propanol (n-propanol and/or isopropanol),
(b) 0.5 to 10 wt.% of a surfactant selected from sulfate salt surfactants, preferably a sulfate salt surfactant comprising (i) an anionic molecule comprising a monovalent hydrocarbon group having 8 to 20 carbon atoms, which is linked via an alkyleneoxy unit or polyalkyleneoxy unit with a sulfate group and (ii) as cationic counterion a protonated amine, and
water.

It is a characteristic feature of the compositions of the invention that they provide antimicrobial and cleansing action at the same time.

As "antimicrobial" we understand the capacity to kill or inhibit the growth of microorganisms selected from bacteria, viruses and fungi.

This antimicrobial cleansing composition is preferably provided as a transparent or translucent aqueous solution. Hence, it normally comprises one single aqueous-alcoholic phase but no further phases, for instance of oil type.

In a further embodiment, this antimicrobial cleansing composition comprises
(a) 20 to less than 60 wt.%, preferably 25 to 55 wt.%, more preferably 35 to 50 wt.% of ethanol containing not more than 20 wt.% propanol (n-propanol and/or isopropanol),
(b) 0.5 to 10 wt.% of the anionic surfactant described above,
(b') up to 5 wt.%, preferably up to 1 wt.% of a secondary surfactant (different from surfactant (b)) said secondary surfactant preferably being a nonionic co-surfactant,
(c) optionally up to 5 wt.%, e.g. 0.001 to 4 wt.%, of an agent selected from antibacterial, antifungal and antiviral agents,
(d) optionally 0.001 to 5 wt.%, e.g. 0.1 to 3 wt.%, of a thickener,
(e) optionally 0.001 to 8 wt.%, e.g. 0.5 to 5 wt.%, of a humectant,
(f) optionally 0.001 to 8 wt.%, e.g. 0.5 to 5 wt.%, of a film-forming agent,
(g) optionally 0.001 to 8 wt.%, e.g. 0.1 to 5 wt.%, of at least one skin care active,
(h) optionally 0.001 to 3 wt.%, e.g. 0.1 to 1 wt.%, of a hydrophilic solvent,
(i) optionally 0.001 to 3 wt.%, e.g. up to 1 wt.%, of a pH-regulating compound,
(j) optionally 0.001 to 3 wt.%, e.g. up to 1 wt.%, of other auxiliary substances, and
(k) water.

It should be understood that in the present invention the embodiments given for one of components (a), (b), (b'), (c), (d), (e), (f), (g), (h), (i) and (j), respectively, can be used singly or in combination with other embodiments given for the same component. When one of the embodiments given for these components is used singly (as the only embodiment of component (a), (b), etc. present in the composition), the numerical wt.% ranges disclosed in the present application apply to the singly used component as well.

According to one preferred embodiment, this antimicrobial cleansing composition is capable of forming a foam and comprises at least (a) 35 to 50 wt.% of ethanol containing not more than 20 wt.% propanol, and (b) 1 to 8 wt.% of the surfactant described above. In a further embodiment, this foamable composition comprises further (e) 0.5 to 5 wt.% of a humectant, (g) 0.1 to 5 wt.% of at least one skin care active, and optionally one or more of optional components (c), (f), (h), (i) and (j) in the above-stated amounts, the remainder preferably being water.

The present invention also relates to the use of this antimicrobial cleansing composition for making foam.

Further, the invention relates to a dispenser, preferably a foam-generating dispenser containing the above antimicrobial cleansing compositions.

### Detailed description of the invention

The antimicrobial cleansing composition of the invention comprises
(a) 20 to less than 60 wt.%, preferably 25 to 55 wt.%, more preferably 35 to 50 wt.% of ethanol containing not more than 20 wt.% propanol (n-propanol and/or isopropanol),
(b) 0,5 to 10 wt.% of a surfactant selected from sulfate salt surfactants, preferably a sulfate salt surfactant comprising (i) an anionic molecule comprising a monovalent hydrocarbon group having 8 to 20 carbon atoms, which is linked via an alkyleneoxy unit or polyalkyleneoxy unit with a sulfate group and (ii) as cationic counterion a protonated amine, and
water.

The composition developed by the present inventors shows a unique combination of cleansing and antimicrobial properties. Hence, using this composition it will no longer be required to provide in hospitals two separate dispensers for one (foam) soap and one antimicrobial alcohol-based composition.

Surprisingly, the present inventors found that the use of specific surfactants, e.g. MIPA-C12-14 fatty alcohol polyethyleneglycolether sulfate, in fairly low amounts enhances the antimicrobial effect of alcohol-based disinfectant compositions. This makes it possible to use lower amounts of alcohol than present in conventional antimicrobial cleansing compositions. Both low alcohol and low surfactant content contribute to the mildness of the composition. For this reason, it presents no problem if the user of does not completely rinse off antimicrobial composition remaining on his hands.

Further, surfactant (b) increases the capacity of the antimicrobial cleansing composition to form foam. In some embodiments of the present invention, stable foam can be generated with the use of a non-propellant foam-dispensing device from a non-pressurized container.

According to the present invention, 20 to less than 60 wt.%, e.g. 25 to 55 wt.% of ethanol containing not more than 20 wt.% propanol (n-propanol and/or isopropanol) is used as component (a) together with surfactant (b) to provide the cleansing composition witch antimicrobial properties. Preferably 35 to 50 wit.% ethanol containing not more than 20 wt.% propanol (n-propanol and/or isopropanol) is contained in the composition. In further embodiments, ethanol is contained in amounts of 35 to 45 wt.%, such as 37 to 43 wt.%. The antimicrobial cleansing composition contains not more than 20 wt.% propanol, based on the total amount of component (a).

The antimicrobial cleansing composition also comprises 0,5 to 10 wt.%, preferably 1 to 8 wt.%, e.g. from 2 to 6 wt.% or 3 to 5 wt.%, of surfactant (b) which is selected from sulfate salt surfactants.

The sulfate salt surfactant preferably has the following formula (I):

R¹- (O-alkylene)ₙ-OSO₃⁻Z⁺ (I)

wherein n is zero or an integer (e.g. 1 to 10 on average), preferably 1 to 8, 1 to 6, 1 to 4, each on average.

"Alkylene" is preferably ethylene or propylene.

Z⁺ is any positive counter-ion, for instance an alkali metal ion such as K⁺ or Na⁺, an ammonium group (NH⁴) or a protonated amine. Z⁺ may also be selected from quaternary ammonium ions, preferably from those having not more than 10 carbon atoms. The protonated amine can be a monoalkylamine, dialkylamine or trialkylamine having preferably up to 18, more preferably up to 12 carbon atoms in total. The carbon number per alkyl group is preferably up to 6, e.g. 2 to 4 carbon atoms. The N-substituting alkyl may carry further substituents such as hydroxyl as in hydroxyalkyl (alkanol) residues having preferably up to 6, e.g. 2 to 4 carbon atoms. The protonated amine can be a protonated mono-, di- or trihydroxyalkylamine which is selected e.g. from monoethanolamine, diethanolamine, triethanolamine, monopropanolamine, dipropanolamine and tripropanolamine. The propanol unit is preferably an isopropanol unit as in MIPA, DIPA and TIPA.

R¹ is a monovalent hydrocarbon residue having preferably 8 to 24 carbon atoms, more preferably 10 to 16 carbon atoms, such as 12 to 14 carbon atoms. If the surfactant comprises a mixture of hydrocarbon residues having different chain lengths as in many commercially available surfactants the carbon ranges refer to the average carbon number. The monovalent hydrocarbon group may be substituted and/or unsaturated and/or branched. Preferably, it is a linear hydrocarbon group (alkyl group) which neither includes unsaturation nor carries any substituents.

In one preferred embodiment, the sulfate salt surfactant comprises (i) an anionic molecule comprising a monovalent hydrocarbon group having 8 to 20 carbon atoms, which is linked via an alkyleneoxy unit or polyalkyleneoxy unit with a sulfate group and (ii) as cationic counter-ion a protonated amine as defined above, preferably a protonated mono-, di- or tri-hydroxylalkylamine. The protonated mono-, di- or tri-hydroxylalkylamine is preferably one wherein the alkyl group has 2 to 4 carbon atoms and comprises one hydroxyl group substituent.

It should be understood that the amine typically occurs in protonated form due to proton transfer from the protonated form of the sulfate group present in the anionic molecule portion. The cationic counter-ion present in this preferred embodiment is preferably propane substituted by one (protonated) amino group and one hydroxyl group. The amino group and the hydroxyl group are preferably located on adjacent carbon atoms. Most preferably, the counter-ion is (protonated) 2-hydroxy-1-amino propane (isopropanolamine) as in MIPA-laureth sulfate.

The aforementioned (poly) alkyleneoxy unit is preferably formed by ethyleneoxy and/or propyleneoxy units. Preferably, the (poly)alkyleneoxy unit is a (poly)ethyleneoxy unit having on average up to 10 units, or more preferably up to 6 units.

In a preferred embodiment, the anionic molecule portion of the surfactant comprises a C10 to C16 alkyl group, e.g. C12 to C14 alkyl group (average C number in mixtures) which is linked via a (O-CH₂-CH₂ₙ) unit, wherein the average value of n is 1 to 6, or preferably 1 to 4, to a sulfate group. One commercially available representative embodiment of this class of surfactants is MIPA-laureth sulfate having the formula CH₃ (CH₂)₁₀CH₂ (OCH₂CH₂) ₙOSO₃H•H₂NCH₂C(OH)HCH₃, avg. n=1-4.

The antimicrobial cleansing composition may further comprise one or more components selected from secondary surfactants, antibacterial agents, antiviral agents, antifungal agents, thickening agents, humectants, film-forming agents, skin care actives, hydrophilic solvents, pH-regulating substances and other auxiliary agents typically used in antimicrobial cleansing compositions. Their total amount preferably does not exceed 25 wt.%.

It is possible to add secondary surfactants (co-surfactants) to all embodiments of the present invention. Such secondary surfactants (b'), e.g. non-ionic surfactants, may be present in an optional amount of up to 5 wt.%, preferably in an amount of up to 1 wt.%, e.g. 0,1 to 1 wt.%. Nonionic co-surfactants such as sugar-based surfactants, e.g. glucoside surfactants, may be added to further increase the efficiency of surfactant (b) and/or enhance the spreading behaviour of the antimicrobial composition and/or better the after-feel of the antimicrobial composition on the skin. Sometime it may also be convenient to use a commercially available surfactant mixture which contains, in addition to surfactant (b), smaller amounts of secondary surfactants (b').

In view of the excellent antimicrobial properties of the composition, the presence of such secondary surfactants is not required. Hence, in one aspect of the present invention and embodiments thereof, surfactant (b) is the sole surfactant.

The antimicrobial cleansing composition may also contain, as component (c), an optional amount of up to 5 wt.% of at least one agent selected from antibacterial, antifungal and antiviral agents. Their addition may be contemplated, for instance, if activity against specific bacterial, fungal or viral strains is to be provided. In view of the excellent antimicrobial activity of the claimed composition, however, it is one aspect of the the present invention and embodiments thereof not to use such an agent.

The antimicrobial cleansing composition may contain 0.1 to 3 wt.% of a thickener as component (d), particularly in the second embodiment of the present invention where the ability to form stable foam is not required.

The thickening agents can be selected from agents commonly used in alcohol-based cleansing compositions and cosmetic compositions. Preferably, they belong to the class of hydrocolloids. Suitable thickening agents can be selected from cellulose ethers, polysaccharides and polyacrylates. In one embodiment, the thickener is hydroxypropyl cellulose. However, the use of gelling agents is preferably avoided since commercial alcohol gel products sometimes leave an unpleasant, sticky after-feel.

As a further optional component (e), the antimicrobial cleansing compositions may also contain 0.5 to 5 wt.% of a humectant. The function of humectants is to attract water to the *stratum corneum* of the human skin. Suitable humectants may be selected from cosmetic ingredients typically used for this purpose. Specifically, the humectant may be selected from polyols such glycerol, sugar alcohols such as sorbitol, urea, alpha hydroxy acids such as lactic acid, glycolic acid and hyaluronic acid.

As a further optional component (f), film-forming agents may be added in an amount of 0.5 to 5 wt.%. Film-forming agents are capable of inhibiting transepidermal water loss (TWEL) in the *stratum corneum.* Preferred film-forming agents are ethanol-soluble. More preferred are ethanol-soluble silicones such as dimethicone.

As a further optional component (g), the antimicrobial cleansing composition may contain 0.1 to 5 wt.% of at least one skin care active. The skin care actives are preferably obtained from natural sources (plant extracts). They can be selected, for example, from agents having a skin-soothing, antiphlogistic (reduction of skin irritation), wound-healing, and/or cell-regenerating effect. Examples of suitable skin care actives include betaine (trimethylglycine), allantoin; aloe vera extract; chamomile extract containing azulene and bisabolol; echinacea; dragosantanol; panthenol; liquorice root extract containing 18-glycyrrhetinic acid; lime tree extract containing quercetin and/or glyco-rutin; marigold (calendula oil); phytosterols, optionally ethoxylated (available from Henkel under the tradename "Generol"); chitosan (acetylated chitin); anthocyanidins; gingko leaf extract containing quercetin and rutin; horse chestnut containing quercetin and campherol; vitamins or provitamins such as provitamin B5 or Vitamin E; avocado oil; birch extract; arnica; extract of rose of Sharon or St. John's Wort; teatree oil; cucumber, hops or hamamelis extracts or ingredients. The aforementioned extracts can be used singly or in combination. Likewise, the extracts can be substituted for suitable amounts of one or more of their ingredients, as stated above. In the present invention, the use of betaine is preferred in view of its cell-regenerating effect.

As a further optional component (h), the antimicrobial cleansing composition may also contain hydrophilic solvent in an amount of 0.1 to 1 wt.%. This hydrophilic solvent must be dermatologically acceptable in the amounts used. Further, solvents with humectant properties are preferably not considered as "hydrophilic solvents" in the sense of the present invention but are ascribed to component (e). The alcohol component (a) is also to be distinguished from the hydrophilic solvent (h). Examples of hydrophilic solvents include alkylene glycols and water-soluble polyalkylene glycols such as propylene glycol, butylene glycol or dipropylene glycol.

Hydrophilic solvents do not constitute an essential component of the antimicrobial cleansing composition but can be added to enhance the solubilisation of individual components. Sometimes, hydrophilic solvents are present, for instance in the commercially available formulation of other components used to formulate the antimicrobial cleansing composition.

As a further optional component (i), the antimicrobial cleansing composition may also comprise pH-regulating substances in an amount of up to 1 wt.%. The pH of the antimicrobial cleansing composition is preferably 5.0 to 7.0, or more preferably 5.2 to 6.5. The pH-regulating substance can be used for adjusting the pH to these values. It is preferably selected from dermatologically acceptable organic acids such as citric acid or lactic acid.

As a further optional component (j), the antimicrobial cleansing composition may also contain other auxiliary substances which are sometimes used in alcohol-based antimicrobial compositions such as fragrances or dyes. The total amount of such auxiliary substances preferably should not exceed 1 wt.%.

In a preferred **first embodiment,** the antimicrobial cleansing composition is **capable of forming foam** and comprises at least the following ingredients (a) and (b) in the amounts stated below, and water:
(a) 35 to 50 wt. % of ethanol containing not more than 20 wt.% propanol (n-propanol and/or isopropanol),
(b) 1 to 8 wt.% of a surfactant selected from sulfate salt surfactants, preferably a sulfate salt surfactant comprising (i) an anionic molecule comprising a monovalent hydrocarbon group having 8 to 20 carbon atoms, which is linked via an alkyleneoxy unit or polyalkyleneoxy unit with an anionic group and (ii) as cationic counterion an alkyl group having 2 to 4 carbon atoms and comprising at least one amino group and at least one hydroxyl group substituent.

In one aspect of the first embodiment, the composition further comprises:
b') optionally up to 5 wt.%, preferably up to 1 wt.%, e.g. optionally 0,1 to 1,0 wt.% of a secondary surfactant (different from surfactant (b)) said secondary surfactant preferably being a nonionic co-surfactant,
(c) optionally up to 5 wt.% of an agent selected from antibacterial, antifungal and antiviral agents,
(e) optionally 0.5 to 5 wt.% of a humectant,
(f) optionally 0.5 to 5 wt.% of a film-forming agent,
(g) optionally 0.1 to 5 wt.% of at least one skin care active,
(h) optionally 0.1 to 1 wt.% of a hydrophilic solvent,
(i) optionally up to 1 wt.% of a pH-regulating compound,
(j) optionally up to 1 wt.% of other auxiliary substances, and
(k) water.

Surprisingly, it has been found that this antimicrobial cleansing composition is able to be dispensed as a sufficiently stable foam with the use of non-propellant foam-dispensing devices from non-pressurized containers. It is advantageous to provide the antimicrobial cleansing composition as foam to the user since foams can be spread easily and uniformly over the hands. This contributes to the cleansing efficiency of the composition. Despite the low alcohol content, this embodiment of the present invention also shows an excellent antimicrobial activity.

Regarding the preferred embodiments and/or the amounts of components (a), (b), (b'), (c), (e), (f), (g), (h), (i) and (j), respectively, reference is made to the above description. Component (c) is not required in the first embodiment but may be added to supplement the spectrum of antibacterial and/or antifungal and/or antiviral activity.

Moreover, it is preferred that the antimicrobial cleansing composition of the first embodiment comprises
(a) 35 to 45 wt.% of ethanol containing not more than 20 wt.% propanol (n-propanol and/or isopropanol),
(b) 2 to 6 wt.% of a surfactant as defined in claim 1, 2 or 3,
(b') optionally up to 1 wt.%, e.g. 0,1 to 1 wt.% of a secondary surfactant, said secondary surfactant preferably being a nonionic co-surfactant
(e) 0.5 to 3 wt.% of a humectant, preferably selected from diols or triols such as glycerol,
(g) 0.1 to 5 wt.% of at least one skin care active, preferably selected from cell-regenerating agents such as betaine,
(h) optionally 0.1 to 1 wt.% of a hydrophilic solvent,
(i) optionally up to 1 wt.% of a pH-regulating compound, preferably selected from cosmetically acceptable organic acids such as citric acid,
(j) optionally up to 1 wt.% of other auxiliary substances selected from perfumes and dyes,
the remainder preferably being water.

In a further embodiment (the **second embodiments**), the present invention provides an antimicrobial cleansing composition comprising
(a) 30 to 60 wt.% of ethanol containing not more than 20 wt.% propanol (n-propanol and/or isopropanol),
(b) 0.5 to 10 wt.% of a surfactant as defined above and in claims 1, 2 or 3,
(b') up to 5 wt.%, preferably up to 1 wt.%, e.g. 0,1 to 1 wt.% of a secondary surfactant (different from surfactant (b)) said secondary surfactant preferably being a nonionic co-surfactant
(c) optionally up to 5 wt.% of an agent selected from antibacterial, antifungal and antiviral agents,
(d) 0.1 to 3 wt.% of a thickener,
(e) optionally 0.5 to 5 wt.% of a humectant,
(f) optionally 0.5 to 5 wt.% of a film-forming agent,
(g) optionally 0.1 to 5 wt.% of at least one skin care active,
(h) optionally 0.1 to 1 wt.% of a hydrophilic solvent,
(i) optionally up to 1 wt.% of a pH-regulating compound,
(j) optionally up to 1 wt.% of other auxiliary substances, and
(k) water.

During hand wash also this composition generates foam, albeit to a lesser degree than the first embodiment of the invention.

Regarding the preferred embodiments of these components and/or the preferred amounts thereof, reference is made to the above description.

This composition is not suitable to be dispensed as foam in view of the fact that it contains thickener. The thickener is used to adjust the viscosity of this liquid cleansing composition to 100 to 20,000 mPa·s (measured at 23°C with a Brookfield-LV viscosimeter equipped with a helipath, spindle T-F, 12 rpm) .

The present invention also relates to the use of the antimicrobial cleansing composition according to the first embodiment for making foam.

A further aspect of the invention relates to a dispenser containing the antimicrobial cleansing composition according to the invention. This dispenser is preferably a foam dispenser if the foamable antimicrobial composition (the first embodiment of the invention) is to be dispensed. Conventional foam dispensers can be used for this purpose.

A suitable kind of conventional foam dispenser comprises a dispensing assembly to be mounted on or in an opening of a container for holding a liquid to be dispensed in the form of a foam, i.e. the foamable antimicrobial composition (the first embodiment of the invention). The dispensing assembly includes a pump assembly which in turn comprises a liquid piston pump and an air piston pump concentrically arranged in relation to each other. The cylinders of the liquid pump and the air pump are formed by the inner cylinder and outer cylinder of a double cylinder, respectively. A liquid piston and an air piston are reciprocally movably arranged in the liquid cylinder and the air cylinder. The dispensing assembly further comprises a common actuation member for simultaneous actuation of the liquid pump and the air pump by manual depression of the actuation member: the liquid piston and the air piston are operatively connected to the actuation member so that upon actuation of the actuation member the liquid piston and the air piston are reciprocally moved in the liquid cylinder and the air cylinder, respectively.

When the above dispensing assembly is mounted on the container containing a foamable liquid, i.e. the foamable antimicrobial composition according to the first embodiment of the invention, and the actuation member is operated, the liquid and air are compressed in the pump chambers of the liquid pump and the air pump, respectively, and both the liquid and the air are pumped into a mixing chamber to be mixed with each other to form a (pre)foam. The mixture of liquid and air is further pumped through a dispensing channel while passing one or more porous elements, such as sieves, to form a homogeneous foam which is dispensed at a dispensing opening at the end of the dispensing channel.

When the common actuation member is released, the upward stroke of the pump pistons increases the size of the pump chambers so that liquid is drawn from the reservoir into the liquid pump chamber and air is sucked into the air pump chamber. A new amount of foam can now be dispensed by operating the actuating member.

Foam-dispensing assemblies of the type described above are, for instance, described in EP 0 613 728 A2, US 6,053,364, US 2009/0236371 A1 and US2010/0320232 A1, the latter three documents being in the name of REXAM AIRSPRAY N.V., and shown in particular in Fig. 1 of US 2009/0236371 A1, for example. However, it should be understood that in accordance with the present invention, the foam dispenser does not require the porous matrix containing a dispensate material being soluble or dispersible in the already generated foam which is present in the dispenser of Fig. 1 of US 2009/0236371 A1.

Dispensing assemblies of this type are commercially available from REXAM AIRSPRAY N.V. and are suitable for use with the present invention.

### Examples

The following examples illustrate the present invention.

### Example 1 - antimicrobial cleansing composition

A foamable antimicrobial cleansing composition was prepared at room temperature by mixing water and alcohol and dissolving the remaining components in the mixture.
▪ 40 wt.% denatured alcohol (36 wt.% ethanol/4 wt.% isopropyl alcohol as denaturation agent)
▪ 4.0 wt.% of a solution of 85 wt.% MIPA-C12-14 fatty alcohol polyethyleneglycolether sulfate and 5 wt.% ethoxylated C12-C14 fatty alcohols in 10 wt.% propylene glycol (Marlinat® 242/90 from Sasol Germany GmbH O&S)
▪ 1 wt.% glycerin
▪ 1 wt.% betaine
▪ ≤ 0.5 wt.% citric acid (to adjust the pH to 5 to 6)
▪ ad 100 wt.% aqua

After mixing a transparent aqueous solution was obtained.

Using a conventional non-propellant foam-dispensing device, this antimicrobial cleansing composition yielded a foam which was sufficiently stable (at least for about 20 seconds) for washing the hands.

This was unexpected since MIPA-laureth sulfate is typically used in water-free formulations (e.g. bath oils) and is known for having good perfume and oil emulsifying characteristics which are not related to the effects achieved by the present invention.

This antimicrobial foam cleanser was evaluated in various tests as follows:

In an epicutaneous 24 hour patch test with 22 test persons, the skin tolerability of the foam cleanser was found to be very good. The mean irritation scores were within the range of the negative control (aqua demineralised) at all readings.

The bacterial efficacy of the foam cleanser in the quantitative suspension test according to DIN EN 1040 (status December 2005) on the test organisms *Staphylococcus aureus* ATCC 6538 and *Pseudomonas aeruginosa* ATCC 15442 was confirmed (reduction ≥ 5.0 lg).

Further, the antimicrobial foam cleanser was subjected to a hygienic hand wash test according to DIN EN 1499 based on the following wash procedure:
1. Moisten the hands with warm water at 30°C for about 10 seconds.
2. Wash thoroughly for 2 x 30 seconds using 2 x four pump strokes (2 x 1.5 g) of foam cleanser. Massage the soap on both sides of the hands and between the fingers for 60 seconds, in accordance with the standard procedure.
3. Rinse the hands with water at 30°C for 30 seconds.
4. Dry the hands using a paper towel until the skin feels dry.

The pH of the foam cleanser was 5.37 and the test strain *Escherichia. (E.) coli* K12 (NCTC 10538). Using the reference hand wash, the hands were washed with 1 x 5 ml Kalisoap within 1 minute, immediately after the determination of the pre-values. The foam cleanser of the invention showed a mean log reduction of 3.30 log (n=15) compared to a mean log reduction of 2.86 log (n=15) for the reference product. Therefore, the antimicrobial foam cleanser fulfils its function as a product for hygienic hand washing, in accordance with the requirements of EN 1499.

In accordance with the requirements of EN 1276, it was also found that the antimicrobial foam cleanser showed a bacterial efficacy with respect to the tested strains shown below at a concentration of 75% after a contact time of 5 minutes under dirty conditions and at a test temperature of 20°C:

| | | |
|---|---|---|
| *Staphylococcus (S.) aureus* | ATCC | 6538 |
| *Escherichia (E.) coli* | ATCC | 10536 |
| *Enterococcus (Ec.) hirae* | ATCC | 10541 |
| *Pseudomonas (P.) aeruginosa* | ATCC | 15442 |

In the evaluation of the fungicidal activity according to EN 1275, the antimicrobial foam cleanser showed a so-called "yeasticidal" activity with reference to the tested strain *Candida albicans* (ATCC 10231) because it passed the required criteria of > 4.0 log reduction after a contact time of 15 min. The log reduction at the same concentration with reference to *Aspergillus brasiliensis* (ATCC 16404) was however only log 0.44.

The virus-inactivating properties of the antimicrobial foam cleanser against Vaccinia virus and Bovine Viral Diarrhea Virus (BVDV, surrogate of Hepatitis C Virus) were tested with an "undiluted" solution of the foam cleanser resulting from the addition of 20 parts by weight test virus suspension to 80 parts by weight foam cleanser. It was found that an exposure time of 30 seconds was required in order to achieve a four log₁₀ reduction (inactivation ≥ 99.99%) in a quantitative suspension test according to the guideline of the Deutsche Vereinigung zur Bekämpfung der Viruskrankheiten e.V. (DVV, German Association for the Control of Virus Diseases) and the Robert-Koch-Institute (RKI).

After evaluation with Vaccinia virus and BVDV, the antimicrobial Foam Cleanser can be declared as having "limited virucidal" properties according to a recommendation of an expert committee of RKI (Bundesgesundheitsbl 2004, 47:62-66) and is thus able to inactivate all enveloped viruses.

Therefore, after successful experiments with the two above mentioned enveloped viruses the antimicrobial Foam Cleanser is also effective against the so-called blood-born viruses including HBV, HCV and HIV as well as against members of other virus families such as orthomyxoviridae (incl. all human and animal influenza viruses like H5N1 and H1N1).

### Examples 2 to 4 and Reference Examples 5 and 6

The antimicrobial cleansing composition of Example 1 was modified by replacing 4.0 wt.% of Marlinat® 242/90 by 2.0 wit.% of one of the following surfactants:
▪ Example 2: Mackadet® from Rhodia UK Ltd., i.e. MIPA-laureth sulphate (33-39 wt.%), laureth-3 (32-38wt.%) and cocamide MIPA (12-16wt.%) dissolved in butylene glycol and propylene glycol.
▪ Example 3: Sodium laureth sulphate
▪ Example 4: Sodium lauryl sulfate
▪ Reference Example 5: Cocamidopropyl betaine
▪ Preference Example 6: Sodium cocoamphoacetate

These compositions resulted in less stable but still acceptable foams when compared with example 1. The foam of Example 2 was almost as good as that of example 1, followed by the foams of example 3 and 4, respectively.

### Comparative Examples 1, 2 and 3

The antimicrobial cleansing composition of Example 1 was modified by replacing 4.0 wt.% of Marlinat® 242/90 by 2.0 wt.% of one of the following surfactants:
▪ Comparative Example 1: Coco glucoside and disodium lauryl sulfosuccinate
▪ Comparative Example 2: Coco glucoside
▪ Comparative Example 3: Lauramine oxide

The stability of the foams generated by these comparative products was rated poor.

## Claims

1. An antimicrobial cleansing composition comprising
(a) 20 to less than 60 wt.% of ethanol containing not more than 20 wt.% n-propanol and/or isopropanol,
(b) 0,5 to 10 wt.% of a surfactant selected from sulfate salt surfactants, and
water.

2. An antimicrobial cleansing composition according to claim 1, wherein surfactant (b) is a sulfate salt surfactant and comprises (i) an anionic molecule comprising a monovalent hydrocarbon group having 8 to 20 carbon atoms, which is linked via an alkyleneoxy unit or polyalkyleneoxy unit with a sulfate group and (ii) as cationic counterion a protonated amine.

3. An antimicrobial cleansing composition according to claim 1 or 2, wherein the cationic counter-ion of the surfactant comprises propane substituted by one amino group and one hydroxyl group.

4. An antimicrobial cleansing composition according to claim 1, 2 or 3, further comprising one or more components selected from secondary surfactants, antibacterial agents, antiviral agents, antifungal agents, thickening agents, humectant agents, film-forming agents, skin care actives, hydrophilic solvents, pH-regulating substances and other auxiliary agents.

5. An antimicrobial cleansing composition according to claim 1, 2, 3 or 4 having a pH of 5 to 7.

6. An antimicrobial cleansing composition according to claim 1, 2, 3, 4 or 5 further comprising
(b') optionally up to 5 wit.% of a secondary surfactant,
(c) optionally up to 5 wt.% of an agent selected from antibacterial, antifungal and antiviral agents,
(d) optionally 0.1 to 3 wt.% of a thickener,
(e) optionally 0.5 to 5 wt.% of a humectant agent,
(f) optionally 0.5 to 5 wt.% of a film-forming agent,
(g) optionally 0.1 to 5 wt.% of at least one skin care active,
(h) optionally 0.1 to 3 wt.% of a hydrophilic solvent,
(i) optionally up to 1 wt.% of a pH-regulating compound,
(j) optionally up to 1 wt.% of other auxiliary substances.

7. An antimicrobial cleansing composition according to claim 1, 2, 3, 4, 5 or 6, wherein
(d) the thickener is selected from cellulose ethers,
(e) the humectant agent is selected from polyols such as glycerol,
(f) the film-forming agent is selected from ethanol-soluble oils such as silicones,
(g) the skin care active is selected from cell-regenerating agents such as betaine,
(i) the pH-regulating compound is selected from organic acids such as citric acid, and
(j) other auxiliary substances are selected from perfumes and dyes.

8. An antimicrobial cleansing composition according to claim 1, 2, 3, 4, 5, 6 or 7, comprising
(a) 35 to 50 wt.% of ethanol containing not more than 20 wt.% n-propanol and/or isopropanol,
(b) 1 to 8 wt.% of a surfactant as defined in claim 1, 2 or 3,
(b') optionally up to 5 wit.% of a secondary surfactant,
(c) optionally up to 5 wt.% of an agent selected from antibacterial, antifungal and antiviral agents,
(e) 0.5 to 5 wt.% of a humectant agent,
(f) optionally 0.5 to 5 wt.% of a film-forming agent,
(g) 0.1 to 5 wt.% of at least one skin care active,
(h) optionally 0.1 to 3 wt.% of a hydrophilic solvent,
(i) optionally up to 1 wt.% of a pH-regulating compound,
(j) optionally up to 1 wt.% of other auxiliary substances.

9. An antimicrobial cleansing composition according to claim 8, comprising
(a) 35 to 45 wt.% of ethanol containing not more than 20 wt.% n-propanol and/or isopropanol,
(b) 2 to 6 wt.% of a surfactant as defined in claim 1, 2 or 3,
(b') optionally up to 1 wit.% of a secondary surfactant,
(e) 0.5 to 3 wt.% of a humectant,
(g) 0.1 to 5 wt.% of at least one skin care active,
(h) optionally 0.1 to 1 wt.% of a hydrophilic solvent,
(i) optionally up to 1 wt.% of a pH-regulating compound,
(j) optionally up to 1 wt. % of other auxiliary substances selected from perfumes and dyes.

10. An antimicrobial cleansing composition according to claim 1, 2, 3, 4, 5, 6 or 7, further comprising
(b') optionally up to 5 wt.% of a secondary surfactant,
(c) optionally up to 5 wt.% of an agent selected from antibacterial, antifungal and antiviral agents,
(d) 0.1 to 3 wt.% of a thickener,
(e) 0.5 to 5 wt.% of a humectant,
(f) optionally 0.5 to 5 wt.% of a film-forming agent,
(g) 0.1 to 5 wt.% of at least one skin care active,
(h) optionally 0.1 to 3 wt.% of a hydrophilic solvent,
(i) optionally up to 1 wt.% of a pH-regulating compound,
(j) optionally up to 1 wt.% of other auxiliary substances.

11. An antimicrobial cleansing composition according to claim 10, wherein the thickener is selected from cellulose ether and the composition has a viscosity of 100 to 20000 measured with a Brookfield LV viscosimeter at 23°C, spindle T-F, 12 rpm.

12. Use of the antimicrobial cleansing composition of claim 8 or 9 for making a foam.

13. A dispenser containing the antimicrobial cleansing composition of any of claims 1 to 11.

14. A dispenser according to claim 13 which is a foam soap dispenser containing the antimicrobial cleansing composition of claim 8 or 9.

15. A dispenser according to claim 14, comprising a container for accommodating the antimicrobial cleansing composition and a dispensing assembly to be mounted on or in an opening of the container,
wherein the dispensing assembly includes a pump assembly comprising a liquid piston pump and an air piston pump, and a common actuation member for simultaneously actuating the liquid pump and the air pump,
and wherein the pump assembly includes a mixing chamber for mixing the antimicrobial composition with air, and a dispensing channel which includes one or more porous elements for forming a homogeneous foam from the mixture and a dispensing opening for dispensing the foam.

## Patentansprüche

1. Antimikrobielle Reinigungszusammensetzung, umfassend
(a) 20 bis weniger als 60 Gew.% Ethanol, das nicht mehr als 20 Gew.% n-Propanol und/oder Isopropanol enthält,
(b) 0,5 bis 10 Gew.% eines Tensids, ausgewählt aus Sulfatsalztensiden, und
Wasser.

2. Antimikrobielle Reinigungszusammensetzung gemäß Anspruch 1, wobei das Tensid (b) ein Sulfatsalztensid ist und (i) ein anionisches Molekül mit einer monovalenten Kohlenwasserstoffgruppe mit 8 bis 20 Kohlenstoffatomen, die über eine Alkylenoxyeinheit oder Polyalkylenoxyeinheit mit einer Sulfatgruppe verbunden ist, und (ii) als kationisches Gegenion ein protoniertes Amin umfasst.

3. Antimikrobielle Reinigungszusammensetzung gemäß Anspruch 1 oder 2, wobei das kationische Gegenion des Tensids Propan, das durch eine Aminogruppe und eine Hydroxylgruppe substituiert ist, umfasst.

4. Antimikrobielle Reinigungszusammensetzung gemäß Anspruch 1, 2 oder 3, die ferner ein oder mehrere Bestandteile, die aus sekundären Tensiden, antibakteriellen Mitteln, antiviralen Mitteln, Antipilzmitteln, Verdickungsmitteln, Befeuchtungsmitteln, filmbildenden Mitteln, Hautpflegewirkstoffen, hydrophilen Lösungsmitteln, pH-regulierenden Substanzen und anderen Hilfsstoffen ausgewählt sind, umfasst.

5. Antimikrobielle Reinigungszusammensetzung gemäß Anspruch 1, 2, 3 oder 4, die einen pH-Wert von 5 bis 7 hat.

6. Antimikrobielle Reinigungszusammensetzung gemäß Anspruch 1, 2, 3, 4 oder 5, die ferner umfasst:
(b') wahlweise bis zu 5 Gew.% eines sekundären Tensids,
(c) wahlweise bis zu 5 Gew.% eines Mittels, ausgewählt aus antibakteriellen, Antipilz- und antiviralen Mitteln,
(d) wahlweise 0,1 bis 3 Gew.% eines Verdickungsmittels,
(e) wahlweise 0,5 bis 5 Gew.% eines Befeuchtungsmittels,
(f) wahlweise 0,5 bis 5 Gew.% eines filmbildenden Mittels,
(g) wahlweise 0,1 bis 5 Gew.% von mindestens einem Hautpflegewirkstoff,
(h) wahlweise 0,1 bis 3 Gew.% eines hydrophilen Lösungsmittels,
(i) wahlweise bis zu 1 Gew.% einer pH-regulierenden Verbindung und
(j) wahlweise bis zu 1 Gew.% an anderen Hilfsstoffen.

7. Antimikrobielle Reinigungszusammensetzung gemäß Anspruch 1, 2, 3, 4, 5 oder 6, wobei
(d) das Verdickungsmittel aus Celluloseethern ausgewählt ist,
(e) das Befeuchtungsmittel aus Polyolen, wie Glycerin, ausgewählt ist,
(f) das filmbildende Mittel aus Ethanol-löslichen Ölen, wie Silikonen, ausgewählt ist,
(g) der Hautpflegewirkstoff aus zellregenerierenden Mitteln, wie Betain, ausgewählt ist,
(i) die pH-regulierende Verbindung aus organischen Säuren, wie Zitronensäure, ausgewählt ist und
(j) die anderen Hilfsstoffe aus Parfümen und Farbstoffen ausgewählt sind.

8. Antimikrobielle Reinigungszusammensetzung gemäß Anspruch 1, 2, 3, 4, 5, 6 oder 7, umfassend
(a) 35 bis 50 Gew.% Ethanol, das nicht mehr als 20 Gew.% n-Propanol und/oder Isopropanol enthält,
(b) 1 bis 8 Gew.% eines Tensids, wie in Anspruch 1, 2 oder 3 definiert,
(b') wahlweise bis zu 5 Gew.% eines sekundären Tensids,
(c) wahlweise bis zu 5 Gew.% eines Mittels, ausgewählt aus antibakteriellen, Antipilz- und antiviralen Mitteln,
(e) 0,5 bis 5 Gew.% eines Befeuchtungsmittels,
(f) wahlweise 0,5 bis 5 Gew.% eines filmbildenden Mittels,
(g) 0,1 bis 5 Gew.% von mindestens einem Hautpflegewirkstoff,
(h) wahlweise 0,1 bis 3 Gew.% eines hydrophilen Lösungsmittels,
(i) wahlweise bis zu 1 Gew.% einer pH-regulierenden Verbindung und
(j) wahlweise bis zu 1 Gew.% an anderen Hilfsstoffen.

9. Antimikrobielle Reinigungszusammensetzung gemäß Anspruch 8, umfassend:
(a) 35 bis 45 Gew.% Ethanol, das nicht mehr als 20 Gew.% n-Propanol und/oder Isopropanol enthält,
(b) 2 bis 6 Gew.% eines Tensids, wie in Anspruch 1, 2 oder 3 definiert,
(b') wahlweise bis zu 1 Gew.% eines sekundären Tensids,
(e) 0,5 bis 3 Gew.% eines Befeuchtungsmittels,
(g) 0,1 bis 5 Gew.% von mindestens einem Hautpflegewirkstoff,
(h) wahlweise 0,1 bis 1 Gew.% eines hydrophilen Lösungsmittels,
(i) wahlweise bis zu 1 Gew.% einer pH-regulierenden Verbindung und
(j) wahlweise bis zu 1 Gew.% an anderen Hilfsstoffen, ausgewählt aus Parfümen und Farbstoffen.

10. Antimikrobielle Reinigungszusammensetzung gemäß Anspruch 1, 2, 3, 4, 5, 6 oder 7, ferner umfassend:
(b') wahlweise bis zu 5 Gew.% eines sekundären Tensids,
(c) wahlweise bis zu 5 Gew.% eines Mittels, ausgewählt aus antibakteriellen, Antipilz- und antiviralen Mitteln,
(d) 0,1 bis 3 Gew.% eines Verdickungsmittels,
(e) 0,5 bis 5 Gew.% eines Befeuchtungsmittels,
(f) wahlweise 0,5 bis 5 Gew.% eines filmbildenden Mittels,
(g) 0,1 bis 5 Gew.% von mindestens einem Hautpflegewirkstoff,
(h) wahlweise 0,1 bis 3 Gew.% eines hydrophilen Lösungsmittels,
(i) wahlweise bis zu 1 Gew.% einer pH-regulierenden Verbindung und
(j) wahlweise bis zu 1 Gew.% an anderen Hilfsstoffen.

11. Antimikrobielle Reinigungszusammensetzung gemäß Anspruch 10, wobei das Verdickungsmittel aus Celluloseether ausgewählt ist und die Zusammensetzung eine Viskosität von 100 bis 20.000, gemessen mit einem Brookfield-LV-Viskosimeter bei 23°C, Spindel T-F, 12 UPM, aufweist.

12. Verwendung der antimikrobiellen Reinigungszusammensetzung gemäß Anspruch 8 oder 9 zur Herstellung eines Schaums.

13. Spender, der die antimikrobielle Reinigungszusammensetzung gemäß einem der Ansprüche 1 bis 11 enthält.

14. Spender gemäß Anspruch 13, der ein Schaumseifenspender mit der antimikrobiellen Reinigungszusammensetzung gemäß Anspruch 8 oder 9 ist.

15. Spender gemäß Anspruch 14, der einen Behälter zur Aufnahme der antimikrobiellen Reinigungszusammensetzung und eine Abgabeeinheit, die auf oder in einer Öffnung des Behälters montiert ist, umfasst,
wobei die Abgabeeinheit eine Pumpeneinheit mit einer Flüssigkolbenpumpe und eine Luftkolbenpumpe und ein gemeinsames Betätigungselement zur gleichzeitigen Betätigung der Flüssigkeitspumpe und der Luftpumpe umfasst, und
wobei die Pumpeneinheit eine Mischkammer zum Mischen der antimikrobiellen Zusammensetzung mit Luft und einen Abgabekanal, der ein oder mehrere poröse Elemente zur Bildung eines homogenen Schaums aus der Mischung aufweist, und eine Abgabeöffnung zur Abgabe des Schaums umfasst.

## Revendications

1. Composition nettoyante antimicrobienne, comprenant :
a) de 20 à moins de 60 % en poids d'éthanol ne contenant pas plus de 20 % en poids de n-propanol et/ou d'isopropanol,
b) de 0,5 à 10 % en poids d'un tensioactif choisi parmi les tensioactifs de type sel sulfate,
et de l'eau.

2. Composition nettoyante antimicrobienne conforme à la revendication 1, dans laquelle le tensioactif (b) est un tensioactif de type sel sulfate et comprend
i) une molécule anionique comprenant un groupe hydrocarboné monovalent comportant 8 à 20 atomes de carbone, qui est raccordé, par l'intermédiaire d'un motif alcanediyl-oxy ou poly(alcanediyl-oxy), à un groupe sulfate,
ii) et en tant que contre-ion cationique, une amine protoriée.

3. Composition nettoyante antimicrobienne conforme à la revendication 1 ou 2, dans laquelle le contre-ion cationique du tensioactif comprend une molécule de propane portant, en tant que substituants, un seul groupe amino et un seul groupe hydroxyle.

4. Composition nettoyante antimicrobienne conforme à la revendication 1, 2 ou 3, comprenant en outre un ou plusieurs composant(s) choisi(s) parmi les tensioactifs secondaires, agents antibactériens, agents antiviraux, agents antifongiques, agents épaississants, agents humectants, agents filmogènes, substances actives pour soins de la peau, solvants hydrophiles, substances régulant le pH, et autres agents auxiliaires.

5. Composition nettoyante antimicrobienne conforme à la revendication 1, 2, 3 ou 4, présentant un pH de 5 à 7.

6. Composition nettoyante antimicrobienne conforme à la revendication 1, 2, 3, 4 ou 5, comprenant en outre
b') en option, jusqu'à 5 % en poids d'un tensioactif secondaire,
c) en option, jusqu'à 5 % en poids d'un agent choisi parmi les agents antibactériens, antifongiques ou antiviraux,
d) en option, de 0,1 à 3 % en poids d'un épaississant,
e) en option, de 0,5 à 5 % en poids d'un agent humectant,
f) en option, de 0,5 à 5 % en poids d'un agent filmogène,
g) en option, de 0,1 à 5 % en poids d'au moins une substance active pour soins de la peau,
h) en option, de 0,1 à 3 % en poids d'un solvant hydrophile,
i) en option, jusqu'à 1 % en poids d'un composé régulant le pH,
j) et, en option, jusqu'à 1 % en poids d'autres substances auxiliaires.

7. Composition nettoyante antimicrobienne conforme à la revendication 1, 2, 3, 4, 5 ou 6, dans laquelle
d) l'épaississant est choisi parmi les éthers de cellulose,
e) l'agent humectant est choisi parmi les polyols, comme le glycérol,
f) l'agent filmogène est choisi parmi les huiles solubles dans l'éthanol, comme les silicones,
g) la substance active pour soins de la peau est choisie parmi les agents de régénération des cellules, comme la bétaïne,
i) le composé régulant le pH est choisi parmi les acides organiques, comme l'acide citrique,
j) et les autres substances auxiliaires sont choisies parmi les parfums et les colorants.

8. Composition nettoyante antimicrobienne conforme à la revendication 1, 2, 3, 4, 5, 6 ou 7, comprenant
a) de 35 à 50 % en poids d'éthanol ne contenant pas plus de 20 % en poids de n-propanol et/ou d'isopropanol,
b) de 1 à 8 % en poids d'un tensioactif tel que défini dans la revendication 1, 2 ou 3,
b') en option, jusqu'à 5 % en poids d'un tensioactif secondaire,
c) en option, jusqu'à 5 % en poids d'un agent choisi parmi les agents antibactériens, antifongiques ou antiviraux,
e) de 0,5 à 5 % en poids d'un agent humectant,
f) en option, de 0,5 à 5 % en poids d'un agent filmogène,
g) de 0,1 à 5 % en poids d'au moins une substance active pour soins de la peau,
h) en option, de 0,1 à 3 % en poids d'un solvant hydrophile,
i) en option, jusqu'à 1 % en poids d'un composé régulant le pH,
j) et, en option, jusqu'à 1 % en poids d'autres substances auxiliaires.

9. Composition nettoyante antimicrobienne conforme à la revendication 8, comprenant
a) de 35 à 45 % en poids d'éthanol ne contenant pas plus de 20 % en poids de n-propanol et/ou d'isopropanol,
b) de 2 à 6 % en poids d'un tensioactif tel que défini dans la revendication 1, 2 ou 3,
b') en option, jusqu'à 1 % en poids d'un tensioactif secondaire,
e) de 0,5 à 3 % en poids d'un agent humectant,
g) de 0,1 à 5 % en poids d'au moins une substance active pour soins de la peau,
h) en option, de 0,1 à 1 % en poids d'un solvant hydrophile,
i) en option, jusqu'à 1 % en poids d'un composé régulant le pH,
j) et, en option, jusqu'à 1 % en poids d'autres substances auxiliaires, choisies parmi les parfums et les colorants.

10. Composition nettoyante antimicrobienne conforme à la revendication 1, 2, 3, 4, 5, 6 ou 7, comprenant en outre
b') en option, jusqu'à 5 % en poids d'un tensioactif secondaire,
c) en option, jusqu'à 5 % en poids d'un agent choisi parmi les agents antibactériens, antifongiques ou antiviraux,
d) de 0,1 à 3 % en poids d'un épaississant,
e) de 0,5 à 5 % en poids d'un agent humectant,
f) en option, de 0,5 à 5 % en poids d'un agent filmogène,
g) de 0,1 à 5 % en poids d'au moins une substance active pour soins de la peau,
h) en option, de 0,1 à 3 % en poids d'un solvant hydrophile,
i) en option, jusqu'à 1 % en poids d'un composé régulant le pH,
j) et, en option, jusqu'à 1 % en poids d'autres substances auxiliaires.

11. Composition nettoyante antimicrobienne conforme à la revendication 10, dans laquelle l'épaississant est choisi parmi les éthers de cellulose et laquelle composition présente une viscosité, mesurée à l'aide d'un viscosimètre Brookfield LV à 23 °C, avec une broche T-F et à 12 t/min, de 100 à 20 000.

12. Utilisation d'une composition nettoyante antimicrobienne conforme à la revendication 8 ou 9 pour en faire une mousse.

13. Distributeur contenant une composition nettoyante antimicrobienne conforme à l'une des revendications 1 à 11.

14. Distributeur conforme à la revendication 13, qui est un distributeur de savon mousse contenant une composition nettoyante antimicrobienne conforme à la revendication 8 ou 9.

15. Distributeur conforme à la revendication 14, comprenant un récipient destiné à contenir la composition nettoyante antimicrobienne et un dispositif distributeur à monter sur ou dans un orifice du récipient,
lequel dispositif distributeur comporte un groupe de pompage comprenant une pompe à liquide à piston et une pompe à air à piston, et un élément d'entraînement commun qui permet d'actionner simultanément la pompe à liquide et la pompe à air,
et dans lequel le groupe de pompage comporte une chambre de mélange dans laquelle la composition antimicrobienne est mélangée avec de l'air, et un conduit distributeur qui comporte un ou plusieurs élément(s) poreux servant à former une mousse homogène à partir du mélange et un orifice de distribution servant à distribuer la mousse.
